# EUROPEAN PATENT APPLICATION

(11) **EP 2 929 884 A1**
(43) Date of publication of application: **14.10.2015**
(21) Application number: 15162988.8
(22) Date of filing: 09.04.2015
(51) Int. Cl.: A61K 31/4439, A61K 9/20, A61K 9/24, A61K 31/34, A61K 31/415, A61K 31/426, A61K 31/4545, A61P 9/10, A61P 1/00

(54) **PHARMACEUTICAL COMBINATIONS OF DABIGATRAN AND H2-RECEPTOR ANTAGONISTS**

(30) Priority: 11.04.2014 TR 201404232; 09.05.2014 TR 201405225
(71) Applicant: Sanovel Ilac Sanayi ve Ticaret A.S., 34460 Istanbul (TR)
(72) Inventor: Cifter, Ümit, 34460 Istanbul (TR); Türkyilmaz, Ali, 34460 Istanbul (TR); Saydam, Mehtap, 34460 Istanbul (TR); Gülkok, Yildiz, 34460 Istanbul (TR); Kirat Uzunogullari, Nur, 34460 Istanbul (TR); Gökcek, Sevgi, 34460 Istanbul (TR)
(74) Representative: Sevinç, Erkan

(57) **Abstract**

This invention is a novel pharmaceutical composition comprising dabigatran or a pharmaceutically acceptable salt thereof in combination with a H2-receptor antagonist for use in the antithrombotic treatment with preventing or reducing the risk of a gastrointestinal disorder.

## Description

### Field of Invention

This invention is a novel pharmaceutical composition comprising dabigatran or a pharmaceutically acceptable salt in a combination with a H2-receptor antagonist (H2RA) and at least one pharmaceutically acceptable excipient.

More specifically, this invention relates to pharmaceutical composition comprising dabigatran or a pharmaceutically acceptable salt thereof in combination with a H2-receptor antagonist (H2RA) for use in the antithrombotic treatment with preventing or reducing the risk of a gastrointestinal disorder administrated by oral, parenteral, intramuscular or topical route.

### Background of Invention

Thrombotic disorders are characterized by formation of a thrombus that obstructs vascular blood flow locally or detaches and embolizes to occlude blood flow downstream. Thrombotic disorders are a major cause of death in the industrialized world.

Presently there are numerous antithrombotic drugs which are widely available, they also called as Direct Thrombin Inhibitors (DTIs) which are a class of medication that act as anticoagulants (delaying blood clotting) by directly inhibiting the enzyme thrombin. In another exemplary embodiment, the DTI is univalent. In another exemplary embodiment, the DTI is bivalent. In an exemplary embodiment, the DTI is a member selected from hirudin, bivalirudin (IV), lepirudin, desirudin, argatroban (IV), dabigatran, dabigatran etexilate (oral formulation), melagatran, ximelagatran (oral formulation but liver complications) and pro-drugs thereof.

Dabigatran etexilate (Formula I), which is already known from WO 98/37075, is a direct thrombin inhibitor indicated to reduce the risk of stroke and systemic embolism in patients with non-vascular atrial fibrilation.

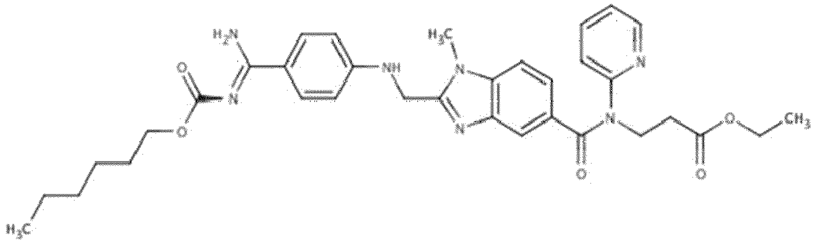

### Formula I: Dabigatran etexilate

The methane sulphonic acid addition salt of dabigatran etexilate, which is commercially available under the trade name PRADAXA®, is disclosed in EP1870100, also disclosed, pellet composition of dabigatran etexilate mesylate (Formula II). This composition is formulated with a core material consisting of organic acid and an active layer which encloses the core.

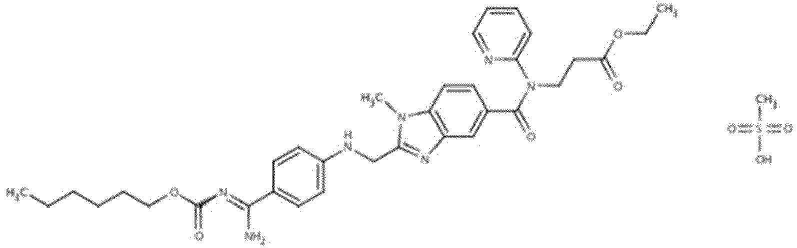

### Formula II: Dabigatran etexilate mesylate

Apart from the mesylate salt of dabigatran etexilate, other acid addition salts of the compound are provided in prior art. For example, WO2012/077136 is directed to the oxalate salt of dabigatran etexilate and besides, its hydrochloride salt is identified in EP1877395. These various form of dabigatran etexilate are prepared to facilitate the development of pharmaceutical composition. An active agent should meet some physicochemical requirements in order to be capable of being used in pharmaceutical compositions. These requirements considerably depend on the physicochemical properties of the active agent used in pharmaceutical composition.

However, dabigatran etexilate is associated with side effects. The administration of antithrombotic agents, such as dabigatran etexilate, have been associated with gastrointestinal disorders such as ulcers and gastrointestinal bleeding. In addition, the administration of dabigatran etexilate, may make some patients more susceptible to the ulcerogenic effects of ulcerogenic stimuli. It appears that a major factor contributing to the development of these gastrointestinal disorders is the presence of acid in the stomach and upper small intestine. While the mechanisms associated with ulcers and gastrointestinal bleeding are not entirely known, there are many causes of ulcers, including stress, alcohol irritation, Helicobacter pylori infection, and the side effects of non-steroid anti-inflammatory drugs. Patients in need of long term antithrombotic drug therapy often may interrupt or not receive such therapy due to gastrointestinal disorders, and as a result, patients are deprived of beneficial antithrombotic drug therapy. There is a need for oral pharmaceutical combination formulations to reduce or eliminate the gastrointestinal disorders associated with use of antithrombotic drugs.

H2-receptor antagonists (H2RA) are competitive, reversible inhibitors of the action of histamine at the histamine H2-receptors found in gastric cells. This results in decreased gastric acid secretion and gastric volume, and reduced hydrogen ion concentration. The acid labile compounds are useful for inhibiting gastric acid secretion.

Famotidine is a histamine H2-receptor antagonist that inhibits stomach acid production. Chemical structure of famotidine (3-[[2-(diaminomethylideneamino)-1,3-thiazol-4-yl]methylsulfanyl]-N'-sulfamoylpropanimidamide) is illustrated with Formula III given below.

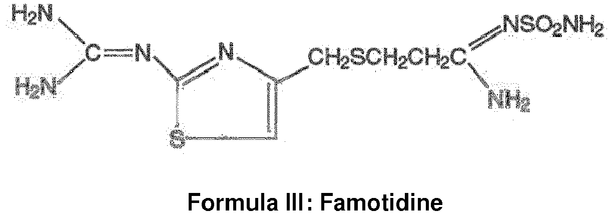

In prior art, US Patent Numbers US4362736 discloses the compound of famotidine. US Patent Numbers US4283408 discloses the medical composition containing famotidine as a gastric acid secretion inhibitor with pharmaceutically acceptable carriers or diluents. Famotidine is commercially available under the trade names PEPCIDINE and PEPCID®. Nizatidine is a histamine H2-receptor antagonist with low toxicity that inhibits stomach acid production and gastric acid secretion. Chemical structure of nizatidine (N-(2-[(2-[(dimethylamino)methyl]thiazol-4-yl)methylthio]ethyl)-N-methyl-2-nitroethene-1,1-diamine) is illustrated with Formula IV given below.

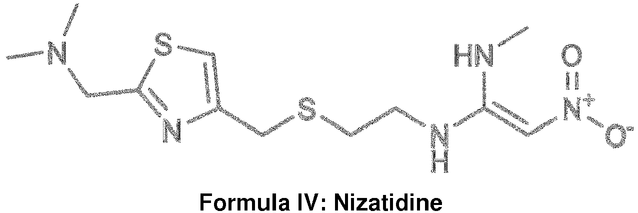

In prior art, US Patent Numbers US4777260 discloses the process for preparing a novel intermediate for nizatidine. US Patent Numbers US4375547 discloses the compound of nizatidine for inhibiting gastric acid secretion in mammals. Nizatidine is commercially available under the trade names TAZAC and AXID®.

Ranitidine is a histamine H2-receptor antagonist that inhibits stomach acid production. Chemical structure of ranitidine (N-(2-[(5-[(dimethylamino)methyl]furan-2-yl)methylthio]ethyl)-N'-methyl-2-nitroethene-1,1-diamine) is illustrated with Formula V given below.

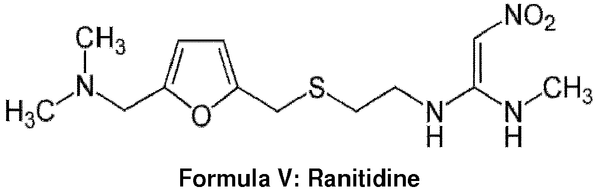

In prior art, US Patent Numbers US4128658 discloses the compound of ranitidine. Additionally ranitidine brings about an improvement of method of treating a condition mediated through histamine H2-receptors, especially for peptic ulceration. US Patent Numbers US5008256 discloses the compound of ranitidine bismuth citrate for treating or preventing gastrointestinal disorders. The bismuth citrate of ranitidine is commercially available under the trade name PYLORID®. EP Patent Numbers EP0626381 discloses a process for preparing form 1 ranitidine hydrochloride. The hydrochloride salt of ranitidine is commercially available under the trade name ZANTAC®.

As described above, the major factor contributing to the development of gastrointestinal disorders is the presence of acid in the stomach and upper small intestine. Therefore, the main object of the invention is administering to a patient an oral dosage form including a combination of an antithrombotic agent and a H2-receptor antagonist (H2RA) which can reduce a major factor contributing to the development of gastrointestinal disorder in patients. According to the main object, the pharmaceutical compositions of this invention is for the prevention gastrointestinal tract from side effects associated with antithrombotic therapy and provide greater antithrombotic effect with the results in a lower incidence of side effects. According to prior inventions dabigatran etexilate in combination with H2-receptor antagonists (H2RA) in particular famotidine or nizatidine or ranitidine have an additive preventive effect in relief of gastrointestinal disorder and provide greater antithrombotic effect with the results in a lower incidence of side effects.

According to the formulation of the present invention it is desired to provide a dosage form comprising in combination a therapeutically effective amount of an antithrombotic agent, specifically dabigatran and a H2-receptor antagonist (H2RA) specifically famotidine or nizatidine or ranitidine which overcomes above described problems. The main challenges when combining those molecules in the same pharmaceutical form are:
(a) to guarantee the physico-chemical compatibility between those different active ingredients and/or between the active ingredients and the excipients used; and
(b) to insure the pharmaceutical compatibility between those active ingredients regarding their stability characteristics. When dabigatran etexilate is combined with H2RA specifically ranitidine, famotidine or nizatidine in order to minimize or prevent the side effects of the dabigatran etexilate, these two drug substances must be released, dissolved and absorbed harmoniously. For the efficiency of the formulation, an easy dissolution of both drug substances at a desired time is an important factor. In order to minimize or prevent the side effects of dabigatran etexilate; it is very important for the molecule, in which the molecule is used in combination with the H2RAs to release both drug substances at the desired time.

### Detailed description of the invention

The present invention relates to a pharmaceutical composition comprising dabigatran or a pharmaceutically acceptable salt, solvate, hydrate, enantiomer, pro-drug or polymorph thereof in a combination with a H2-receptor antagonist (H2RA) or a pharmaceutically acceptable salt, solvate, hydrate, enantiomer, pro-drug or polymorph thereof and at least one pharmaceutically acceptable excipient

The main object of the present invention is to provide new pharmaceutical compositions comprising a specific direct thrombin inhibitor such as dabigatran etexilate in combination with H2-receptor antagonist (H2RA) such as bisfentidine, burimamide, cimetidine, dalcotidine, donetidine, ebrotidine, etintidine, famotidine, lafutidine, lamtidine, lavoltidine (Ioxtidine), lupitidine, metiamide, mifentidine, niperotidine, nizatidine, osutidine, oxmetidine, pibutidine, quisultazine/quisultidine, ramixotidine, ranitidine, ranitidine bismuth citrate, ranitidine hydrochloride, roxatidine, sufotidine, tiotidine, tuvatidine, venritidine, xaltidine or other derivatives or a pharmaceutically acceptable salt, solvate, hydrate, enantiomer, pro-drug or polymorph thereof as effective components which overcomes the above described problems in prior art and have additive advantages over them.

The product in this invention is used for protecting the gastrointestinal tract from side effects associated with antithrombotic therapy using the oral dosage forms described herein. The invention also relates to protect the gastrointestinal tract from side effects associated with antithrombotic therapy by combination an antithrombotic agent and a H2-receptor antagonist (H2RA). These and other features of the present invention are set forth herein more detailed.

In one embodiment, the H2-receptor antagonist (H2RA) is preferably famotidine or nizatidine or ranitidine or a pharmaceutically acceptable salt, solvate, hydrate, enantiomer, pro-drug or polymorph thereof.

In one embodiment, the pharmaceutical composition comprises dabigatran or a pharmaceutically acceptable salt, solvate, hydrate, enantiomer, pro-drug or polymorph thereof and famotidine or a pharmaceutically acceptable salt, solvate, hydrate, enantiomer, pro-drug or polymorph thereof. In a preferred embodiment, famotidine is present in an amount of between 5 mg and 150 mg. In a more preferred embodiment, famotidine is present in an amount of between 10 mg and 100 mg.

In one embodiment, the pharmaceutical composition comprises dabigatran or a pharmaceutically acceptable salt, solvate, hydrate, enantiomer, pro-drug or polymorph thereof and nizatidine or a pharmaceutically acceptable salt, solvate, hydrate, enantiomer, pro-drug or polymorph thereof. In a preferred embodiment, nizatidine is present in an amount of between 10 mg and 500 mg. In a more preferred embodiment, nizatidine is present in an amount of between 50 mg and 400 mg.

In one embodiment, the pharmaceutical composition comprises dabigatran or a pharmaceutically acceptable salt, solvate, hydrate, enantiomer, pro-drug or polymorph thereof and ranitidine or a pharmaceutically acceptable salt, solvate, hydrate, enantiomer, pro-drug or polymorph thereof. In a preferred embodiment, ranitidine is present in an amount of between 5 mg and 500 mg. In a more preferred embodiment, ranitidine is present in an amount of between 10 mg and 450 mg.

In a preferred embodiment, a pharmaceutically acceptable salt, solvate, hydrate, enantiomer, pro-drug or polymorph of dabigatran is the etexilate or etexilate mesylate pro-drugs of dabigatran. In another preferred embodiment dabigatran etexilate is present in an amount of between 50 mg and 250 mg.

In a preferred embodiment dabigatran etexilate or etexilate mesylate present in an amount of between 50 mg and 250 mg and the famotidine or a pharmaceutically acceptable salt, solvate, hydrate, enantiomer, pro-drug or polymorph thereof is present in an amount of between 5 mg and 150 mg.

In a preferred embodiment dabigatran etexilate or etexilate mesylate present in an amount of between 50 mg and 250 mg and the nizatidine or a pharmaceutically acceptable salt, solvate, hydrate, enantiomer, prodrug or polymorph thereof is present in an amount of between 10 mg and 500 mg.

In a preferred embodiment dabigatran etexilate or etexilate mesylate present in an amount of between 50 mg and 250 mg and the ranitidine or a pharmaceutically acceptable salt, solvate, hydrate, enantiomer, prodrug or polymorph thereof is present in an amount of between 5 mg and 500 mg.

According to main object of the invention, pharmaceutical formulations of dabigatran etexilate in a combination with a H2-receptor antagonist (H2RA) have a good stability and have improved manufacturing process that is achieved in a technologically simple way which overcomes the above described problems.

Yet another object of the present invention is to provide an improved process which is simple, cost-effective and time saving for preparing the pharmaceutical composition of dabigatran or pharmaceutically acceptable salts thereof and one or more H2RAs specially

In another embodiment, the oral formulations described herein can be used to treat, prevent or reduce the risk of almost any physiological disorder for which antithrombotic agents are indicated. The methods and formulations provided herein can be administered to any subject in need of therapy including, without limitation, humans, including patients, companion animals, including but not limited to dogs, cats, ferrets, and birds, food-source animals, including, but not limited to cows, pigs, and sheep, and zoo animals, such as monkeys and other primates, and other similar animal species.

In one embodiment, these pharmaceutical combinations are administrated oral, parenteral, intranasal, sublingual, transdermal, transmucosal, ophthalmic, intravenous, pulmonary, intramuscular or rectal administration, and preferably for oral administration. According to this embodiment of the invention, said pharmaceutical composition may be formulated with suitable pharmaceutical diluents, excipients or carriers, suitably selected with respect to a dosage form for oral administration. Examples of dosage forms comprise tablets including compressed tablets, coated or uncoated tablets, multilayer tablets, buccal tablets, sublingual tablets, effervescent compositions, effervescent tablets, immediate release tablets, modified release tablets, film-coated tablets, orally disintegrating tablets, gastric disintegrating tablets, pills, capsules, hard or soft gelatin capsules, powders, mini tablets, pellets, coated bead systems, granules, microspheres, ion exchange resin systems, sterile solutions or suspensions, steril ocular solutions, aerosols, sprays, drops, ampoules, suppositories, ocular systems, parenteral systems, creams, gels, ointments, dragees, sachets; films, orally administrable films, solutions, solids; elixirs, tinctures, suspensions, syrups, colloidal dispersions, dispersions, emulsions and thereof.

Another object of the invention is to provide a pharmaceutical composition for use in antithrombotic treatment and its use in reducing the risk of stroke and systemic embolism in patients with non-vascular atrial fibrilation and protecting the gastrointestinal tract from side effects associated with antithrombotic therapy. Here, H2 receptor antagonist decreases acid level of gastric fluid by inhibiting acid secretion, thereby preventing or minimizing such diseases as gastric erosion, peptic ulcer, major upper gastrointestinal system bleedings, alterations in the intestine permeability and inflammation, all of which are caused by dabigatran.

In a preferred embodiment said pharmaceutical composition is formulated preferably in the form of tablet or capsule or multilayer tablet comprising etexilate or etexilate mesylate pellets of dabigatran and H2-receptor antagonist (H2RA) pellets.

According to the pharmaceutical incompatibility problem, the present invention provides a pharmaceutical composition comprising a inert barrier layer which is present in the middle in such a way that it separates dabigatran etexilate and one of the H2-receptor antagonist (H2RA) layers and the dabigatran etexilate and H2RA layers are present at the outer or inner part of the inert barrier layer. By the virtue of the inert barrier layer, interaction between the dabigatran etexilate and H2RA molecules is prevented and at the same time H2RA molecule is enabled to remain stable in the dosage form. Moreover, another important reason is that the formulations of the active substances with different release properties can be provided in the same form with inert barrier layer. Thus, dabigatran and H2RA molecules are formulated in a way not to interact, and at the same time it is provided that these molecules remain stable in their own form.

In one embodiment of the invention, the pharmaceutical composition may further comprise an inert barrier layer between the two molecules wherein the inert barrier layer is selected from the group comprises starch, lactose, sugar alcohol (D-mannitol, erythritol, etc.), lowsubstituted hydroxypropyl cellulose, hydroxypropyl cellulose, hydroxypropyl methylcellulose, polyvinylpyrrolidone, polyvinyl alcohol, methylcellulose, hydroxyethyl methylcellulose or mixtures thereof. Thus, the multilayer pellet/tablet according to the present invention comprising the dabigatran etexilate and H2RA molecules separately and it is characterized in releasing these molecules together and rapidly. The multilayer pellet/tablet according to the present invention is formulated in such a way that H2RA molecules increase the pH of the stomach and after the release of dabigatran etexilate.

According to the challenges mentioned above the selection of the excipients thus very important. According to this embodiment, one or more pharmaceutically acceptable excipient is selected from the group comprising buffering agents, stabilizers, binders, diluents, dispersing agents, lubricants, glidants, disintegrants, plasticizers, preservatives, sweeteners, flavoring agents, coloring agents, acids or mixtures thereof.

In a preferred embodiment, said pharmaceutical composition comprises an acid which is selected from a group comprising organic acids or inorganic acids or mixtures thereof

Suitable organic acids may comprise but not limited to acetic acid, acrylic acid, adipic acid, aldaric acid, ascorbic acid, azelaic acid, benzoic acid, butyric acid, chloroacetic acid, citric acid, crotonic acid, dimercaptosuccinic acid, formic acid, fumaric acid, gallic acid, glutaric acid, itaconic acid, lactic acid, malic acid, maleic acid, malonic acid, monanoic acid, methacrylic acid, naphtenic acid, oleic acid, oxalic acid, palmitic acid, paracetic acid, propionic acid, pimelic acid, salicylic acid, sebacic acid, succinic acid, stearic acid, sorbic acid, tartaric acid, thiomalic acid, uric acid, glyceric acid, xylonic acid, gluconic acid, neuraminic acid, ulosonic acid, ketodeoxyoctulosonic acid, glucuronic acid, galacturonic acid, iduronic acid, meso-galactaric acid (mucic acid, d-glucaric acid (saccharic acid), glycolic acid, mandelic acid, aspartic acid, glutamic acid, and the like or mixtures thereof.

Organic acids are preferably lactic acid, acetic acid, formic acid, oxalic acid, uric acid, carboxylic acid (adipic acid (dicarboxylic), azelaic acid, butyric acid, crotonic acid, gallic acid, itaconic acid, oleic acid, palmitic acid, propionic acid, sebacic acid, sorbic acid), sugar acid (glyceric acid (3C), xylonic acid (5C), gluconic acid (6C), ulosonic acids), aldaric acids (meso-galactaric acid (mucic acid) (6C), D-glucaric acid (saccharic acid) (6C), mandelic acid) or mixtures thereof.

Suitable inorganic acids may comprise but not limited to hydrogen halides and their solutions:hydrofluoric acid (HF), hydrochloric acid (HCl), hydrobromic acid (HBr), hydroiodic acid (HI); halogen oxoacids: hypochlorous acid (HClO), chlorous acid (HClO2), chloric acid (HClO3), perchloric acid (HClO4), and corresponding compounds for bromine and iodine; sulfuric acid (H2SO4), fluorosulfuric acid (HSO3F), nitric acid (HNO3), phosphoric acid (H3PO4), fluoroantimonic acid (HSbF6), fluoroboric acid (HBF4), hexafluorophosphoric acid (HPF6), chromic acid (H2CrO4), boric acid (H3BO3) and the like or mixtures thereof.

Inorganic acids are preferably hydrogen halides (hydrofluoric acid (HF), hydrobromic acid (HBr), hydroiodic acid (HI)), halogen oxoacids (hypochlorous acid (HClO), and corresponding compounds for bromine and iodine), nitric acid (HNO3), fluoroboric acid (HBF4), chromic acid (H2CrO4) or mixtures thereof.

Suitable buffering agents may comprise but not limited to alkali metal citrate, citric acid/sodium citrate, tartaric acid, fumaric acid, sorbic acid, citric acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate, hydrochloric acid/sodium hydroxide or mixtures thereof, and preferably citric acid, fumaric acid, ascorbic acid, sodium dihydrogen phosphate and the like or mixtures thereof.

Suitable stabilizers may comprise but not limited to citric acid, fumaric acid, tartaric acid, sodium citrate, sodium benzoate, sodium dihydrogen phosphate, calcium carbonate, magnesium carbonate, arginine, lysine, meglamine, tocopherol, butylhydroxyanisole (BHA), butylhydroxytoluene (BHT), ascorbic acid, gallic acid esters or the mixtures thereof, and preferably, citric acid, fumaric acid, arginine and the like or mixtures thereof.

Suitable binders may include but not limited to polyvinylpyrrolidone, polyethylene glycol, polyvinyl alcohol, starch, pregelatinized starch, glucose, glucose syrup, natural gums, sucrose, sodium alginate, cellulose derivatives such as hydroxypropyl methyl cellulose, hydroxypropyl cellulose, carboxy methyl cellulose, methyl cellulose, gelatin, carrageenan, guar gum, carbomer, polymethacrylates, methacrylate polymers, collagens, proteins like gelatin, agar, alginate, alginic acid, xanthan gum, hyaluronic acid, pectin, polysaccharides, carbomer, poloxamer, polyacrylamide, aluminium hydroxide, laponit, bentonit, polyoxyethylene-alkyl ether, polydextrose, polyethylene oxide and the like or mixtures thereof.

Suitable diluents may comprise but not limited to microcrystalline cellulose, mannitol, spray-dried mannitol, lactose, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate and the like or mixtures thereof.

Suitable dispersing agents may comprise but not limited to calcium silicate, magnesium aluminum silicate and the like or mixtures thereof.

Suitable lubricants may comprise but not limited to magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate and the like or mixtures thereof.

Suitable glidants may comprise but not limited to talc, aluminium silicate, colloidal silica, starch and the like or mixtures thereof.

Suitable disintegrants may comprise but not limited to cross-linked polyvinil pyrrolidone (crospovidone), povidone, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate, sodium lauryl sulphate and the like or mixtures thereof.

Suitable plasticizers may comprise but not limited to polyethylene glycols of different molecular weights, propylene glycol and the like or mixtures thereof.

Suitable preservatives may comprise but not limited to methyl paraben, propyl paraben and their salts (such as sodium, potassium), sodium benzoate, citric acid, benzoic acid, butylated hydroxytoluene or butylated hydroxyanisole and the like or mixtures thereof.

Suitable sweeteners may comprise but not limited to aspartame, potassium acesulfame, sodium saccharinate, neohesperidine dihydrochalcone, sucralose, saccharin, sugars such as sucrose, glucose, lactose, fructose or sugar alcohols such as mannitol, sorbitol, xylitol, erythritol and the like or mixtures thereof.

Suitable flavoring agents may comprise but not limited to menthol, peppermint, cinnamon, chocolate, vanillin or fruit essences such as cherry, orange, strawberry, grape, black currant, raspberry, banana, red fruits, wild berries and the like or mixtures thereof.

Suitable coloring agents may comprise but not limited to ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow and the like or mixtures thereof.

In a preferred embodiment of the present invention, said pharmaceutical composition is in the form of tablet or capsule or multilayer tablet comprising etexilate or etexilate mesylate of dabigatran pellets and H2-receptor antagonist (H2RA) pellets and acid pellets which are separate pellets or bilayer or multilayer pellets.

In a preferred embodiment of the present invention said dabigatran pellets comprising preferably methacrylic acid-ethyl acrylate copolymer (Eudragit EPO) or polyvinyl alcohol (PVA) or lactose monohydrate or sugar pellet or polyvinylpyrrolidone (PVP) or dibasic calcium phosphate (DCP) or mixtures thereof as pharmaceutically acceptable excipients

In a preferred embodiment of the present invention said acid pellets comprising preferably adipic acid or sorbic acid or succinic acid or glutaric acid or nitric acid and as an excipient xanthan gum or isopropyl alcohol (IPA) or sodium alginate or microcrystalline cellulose or mannitol or lactose or silicon dioxide or methacrylic acid-ethyl acrylate copolymer or eudragit EPO or polyvinyl alcohol (PVA) or mixtures thereof

In a preferred embodiment of the present invention said h2-receptor antagonist (H2RA) pellets comprising preferably microcrystalline cellulose (MCC) or pregelatinized starch or hydroxypropyl cellulose or yellow iron oxide or croscarmellose sodium or sugar pellets or polyvinylpyrrolidone K30 (PVP K30) or mixtures thereof as pharmaceutically acceptable excipients.

According to the pharmaceutical compositions of the invention may be prepared by conventional technology well known to those skilled in the art such as direct compression, dry granulation, wet granulation and the like. During direct compression, active agent and excipients are mixed, sieved and compressed into dosage forms. During wet granulation, the ingredients are mixed and granulated with a granulation liquid. The granulation process provides agglomerates with a desired homogeneity. The mixture is dried and sieved and optionally mixed with additional excipients. Finally, it is compressed into dosage forms. In addition, this novel pharmaceutical formulation is produced by various technologies such as fluidized bed granulation technique or extrusion/spheronization or spray drying and lyofilization.

### Examples:

### Example-1: Multilayer Core Pelletization (Organic acid pellets are coated with Dabigatran+H2RA pellets)

### Multilayer pellets (organic acid pellets are coated with Dabigatran):

% 5.0 - 95 Adipic acid or Sorbic acid or Succinic acid or Glutaric acid
% 0.05 - 50 Xanthan gum
% 0.05 - 20 Polyvinyl alcohol (PVA) or isopropyl alcohol (IPA)
% 0.05 - 20 Sodium alginate
%15.0 - 60 Dabigatran etexilate mesylate (DEM)
% 0.5 - 40 Methacrylic acid-Ethyl acrylate copolymer (Eudragit EPO) or PVA

### H2RA pellets:

%2.0- 30 Famotidine or Nizatidine or Ranitidine
%15.0- 40 Microcrystalline cellulose (MCC)
%10.0- 50 Pregelatinized starch

### Inert barrier layer:

%0.1- 10 Hydroxypropyl cellulose
%0.1- 10 Yellow iron oxide

### Other Excipients:

%0.1 - 0.2 Silicon dioxide
%0.25 - 2.0 Magnesium stearate

**Production process of multilayer pellets (organic acid pellets are coated with Dabigatran):** Adipic acid or Sorbic acid or Succinic acid or Glutaric acid and Xanthan gum are mixed together. Alcoholic PVA or IPA (isopropyl alcohol) solution is sprayed onto this mixture and pellets produced by fluidized bed pelletizing technique. The pellets are coated with sodium alginate in fluidized bed so acid pellets are manufactured. DEM is dissolved in alcoholic Methacrylic acid-Ethyl acrylate copolymer (Eudragit EPO) or Polyvinyl alcohol (PVA) solution. This solution is sprayed onto the acid pellet by fluidized bed pelletizing technique so multilayer pellets are manufactured.

**Production process of H2RA pellets:** Famotidine or Nizatidine or Ranitidine and microcrystalline cellulose and pregelatinized starch are mixed together then by spraying water a wet mass is formed. Pellets produced from this wet mass by extrusion/spheronization pelletizing technique. A solution/dispersion is prepared by adding hydroxypropyl cellulose and yellow iron oxide as inert barrier layer and the H2RA pellets are coated by spraying this mixture.

The multilayer and coated H2RA pellets first mixed with silicon dioxide and then with magnesium stearate. Finally, the pellets are pressed as tablets or filled into the capsules.

### Example-2: Multilayer Core Pelletization (Dabigatran pellets are coated with organic acid+H2RA pellets)

### Multilayer pellets (Dabigatran pellets are coated with organic acid):

%15.0 - 60 Dabigatran etexilate mesylate (DEM)
%0.5 - 40 Methacrylic acid-Ethyl acrylate copolymer (Eudragit EPO) or Polyvinyl alcohol(PVA)
%5.0 - 40 Lactose monohydrate
%5.0 - 95 Adipic acid or Sorbic acid or Succinic acid or Glutaric acid
% 0.05 - 50 Xanthan gum

### H2RA pellets:

%2.0- 30 Famotidine or Nizatidine or Ranitidine
%15.0- 40 Microcrystalline cellulose (MCC)
%10.0- 50 Pregelatinized starch

### Inert barrier layer:

%0.1- 10 Hydroxypropyl cellulose
%0.1- 10 Yellow iron oxide

### Other Excipients:

%0.1 - 0.2 Silicon dioxide
%0.25 - 2.0 Magnesium stearate

**Production process of multilayer pellets (Dabigatran pellets are coated with organic acid):** DEM and lactose monohydrate are mixed together. Alcoholic Methacrylic acid-Ethyl acrylate copolymer (Eudragit EPO) or Polyvinyl alcohol (PVA) solution is sprayed onto this mixture and pellets produced by fluidized bed pelletizing technique. The solution is prepared by adding adipic acid or sorbic acid or succinic acid or glutaric acid and xanthan gum. This solution is sprayed onto the DEM pellets by fluidized bed pelletizing technique so multilayer pellets are manufactured.

**Production process of H2RA pellets:** Famotidine or Nizatidine or Ranitidine and microcrystalline cellulose and pregelatinized starch are mixed together then by spraying water a wet mass is formed. Pellets produced from this wet mass by extrusion/spheronization pelletizing technique. A solution/dispersion is prepared by adding hydroxypropyl cellulose and yellow iron oxide as inert barrier layer and he H2RA pellets are coated by spraying this mixture in fluidized bed.

The multilayer and coated H2RA pellets first mixed with silicon dioxide and then with magnesium stearate. Finally, the pellets are pressed as tablets or filled into the capsules.

### Example-3: Coated Core Pelletization (Dabigatran pellets+Inorganic acid pellets+H2RA pellets)

### Inorganic acid pellets:

%0.05 - 10 Nitric acid
%5.0 - 40 Microcrystalline cellulose (MCC) or Mannitol or Lactose
%0.1 - 0.2 Silicon dioxide
%0.5 - 40 Methacrylic acid-Ethyl acrylate copolymer (Eudragit EPO) or Polyvinyl alcohol (PVA)

### Dabigatran pellets:

%15.0 - 60 Dabigatran etexilate mesylate (DEM)
%5 - 90 Sugar pellet
%0.1 - 25 Polyvinylpyrrolidone (PVP)
%0.5 - 40 Methacrylic acid-Ethyl acrylate copolymer (Eudragit EPO or Polyvinyl alcohol (PVA)

### H2RA pellets:

%2.0- 30 Famotidine or Nizatidine or Ranitidine
%15.0- 40 Microcrystalline cellulose (MCC)
%10.0- 50 Croscarmellose sodium

### Inert barrier layer:

%0.1- 10 Hydroxypropyl cellulose
%0.1- 10 Yellow iron oxide

### Other Excipients

%0.1 - 0.2 Silicon dioxide
%0.25 - 2.0 Magnesium stearate

**Production Process of inorganic acid pellets:** Microcrystalline cellulose or mannitol or lactose and silicon dioxide are blended, then by spraying dilute nitric acid soution a wet mass is formed by high shear granulator or fluid bed granulator. The pellets are coated by Methacrylic acid-Ethyl acrylate copolymer (Eudragit EPO) or Polyvinyl alcohol (PVA) solution in fluidized bed so inorganic acid pellets are manufactured.

**Production process of Dabigatran pellets** Alcoholic solution/dispersion is prepared by adding DEM and PVP. Sugar pellets are coated with this solution/dispersion. The sugar pellets which were coated with the active ingredient are coated with Methacrylic acid-Ethyl acrylate copolymer (Eudragit EPO) or Polyvinyl alcohol (PVA) and then with PVA solution so DEM pellets are manufactured.

**Production process of H2RA pellets:** Famotidine or Nizatidine or Ranitidine and microcrystalline cellulose and croscarmellose sodium are mixed together then by spraying water a wet mass is formed. Pellets produced from this wet mass by extrusion/spheronization pelletizing technique. A solution/dispersion is prepared by adding hydroxypropyl cellulose and yellow iron oxide as inert barrier layer and the H2RA pellets which were coated with the inert barrier by spraying this mixture.

Coated H2RA pellets and DEM pellets and inorganic acid pellets are mixed. The pellets are first mixed with silicon dioxide and then with magnesium stearate. Finally, the pellets are pressed as tablets or filled into the capsules

### Example-4: Multilayer combination pellet (multilayer pellet production method)

### Multilayer pellets:

H2RA pellets:
   %2.0- 30 Famotidine or Nizatidine or Ranitidine
   %2- 5 Polyvinylpyrrolidone K30 (PVP K30)
   %5.0 - 90 Sugar pellet
Inert barrier layer (inert coated H2RA pellets):
   %0.1- 10 Hydroxypropyl cellulose
   %2- 5 Polyvinylpyrrolidone K30 (PVP K30)
Dabigatran pellets (H2RA pellets are coated with Dabigatran):
   %15.0 - 60 Dabigatran etexilate mesylate (DEM)
   %0.5 - 40 Methacrylic acid-Ethyl acrylate copolymer (Eudragit EPO) or Polyvinyl alcohol (PVA)
   %5.0 - 40 Lactose monohydrate
Acid pellets (H2RA pellets are coated with Dabigatran+Acid):
   %5.0 - 95 Adipic acid or Sorbic acid or Succinic acid or Glutaric acid)
   % 0.05 - 50 Xanthan gum

### Other excipients:

% 0.05 - 20 Polyvinyl alcohol (PVA)
%0.1 - 0.2 Silicon dioxide
%0.25 - 2.0 Magnesium stearate

**Production process multilayer pellets:** A solution/dispersion is prepared by adding Famotidine or Nizatidine or Ranitidine and PVP K30. Sugar pellets are coated with this solution/dispersion. The sugar pellets which were coated with the active ingredient are coated with hydroxypropyl cellulose and PVP K 30 solution/dispersion as inert barrier layer so H2RA pellets are manufactured. DEM and lactose monohydrate are mixed with alcoholic Methacrylic acid-Ethyl acrylate copolymer (Eudragit EPO) or Polyvinyl alcohol (PVA) solution. This mixture is sprayed onto H2RA pellets. The solution is prepared by adding adipic acid or sorbic acid or succinic acid or glutaric acid) and xanthan gum. This solution is sprayed onto the pellets so multilayer pellets are manufactured.

Finally the multilayer pellets are coated by PVA. The multilayer pellets first mixed with silicon dioxide and then with magnesium stearate. The pellets are pressed as tablets or filled into the capsules.

### Example-5: Multilayer combination pellet (multilayer tablet press method)

### Multilayer pellets (Dabigatran pellets are coated with organic acid):

%15.0- 60 Dabigatran etexilate mesylate (DEM)
%0.5- 40 Methacrylic acid-Ethyl acrylate copolymer (Eudragit EPO) or polyvinyl alcohol (PVA)
%5.0- 40 Lactose monohydrate
%5.0 - 95 Adipic acid or Sorbic acid or Succinic acid or Glutaric acid
% 0.05 - 50 Xanthan gum
% 0.05 - 20 PVA

### H2RA pellets:

%2.0- 30 Famotidine or Nizatidine or Ranitidine
%2- 5 Polyvinylpyrrolidone K30 (PVP K30)
%5.0 - 90 Sugar pellet
Inert barrier layer (inert coated H2RA pellets):
%0.1 - 10 Hydroxypropyl cellulose
%2- 5 Polyvinylpyrrolidone K30 (PVP K30)

### Other excipients:

%0.1 - 0.2 Silicon dioxide
%0.25 - 2.0 Magnesium stearate

**Production process of multilayer pellets (Dabigatran pellets are coated with organic acid):** DEM and lactose monohydrate are mixed together. Alcoholic Methacrylic acid-Ethyl acrylate copolymer (Eudragit EPO) or Polyvinyl alcohol(PVA) solution is sprayed onto this mixture and pellets produced by fluidized bed pelletizing technique.The solution is prepared by adding adipic acid or sorbic acid or succinic acid or glutaric acid and xanthan gum.This solution is sprayed onto the DEM pellets by fluidized bed pelletizing technique so multilayer pellets are manufactured

**Production process of H2RA pellets:** A solution/dispersion is prepared by adding Famotidine or Nizatidine or Ranitidine and PVP K30. Sugar pellets are coated with this solution/dispersion. The sugar pellets which were coated with the active ingredient are coated with hydroxypropyl cellulose and PVP K30 solution/dispersion as inert barrier layer so H2RA pellets are manufactured.

The multilayer and H2RA pellets first mixed with silicon dioxide and then with magnesium stearate. Finally, the pellets are pressed by multilayer rotary tablet press machine. Or dry coating-intertwined tablets which have H2RA pellets on the core are produced with special tablet press machine.

## Claims

1. A pharmaceutical composition comprising dabigatran or a pharmaceutically acceptable salt, solvate, hydrate, enantiomer, pro-drug or polymorph thereof in a combination with a H2-receptor antagonist or a pharmaceutically acceptable salt, solvate, hydrate, enantiomer, pro-drug or polymorph thereof and at least one pharmaceutically acceptable excipient.

2. The pharmaceutical composition according to claim 1, the H2-receptor antagonistis selected from a group comprising bisfentidine, burimamide, cimetidine, dalcotidine, donetidine, ebrotidine, etintidine, famotidine, lafutidine, lamtidine, lavoltidine (Ioxtidine), lupitidine, metiamide, mifentidine, niperotidine, nizatidine, osutidine, oxmetidine, pibutidine, quisultazine/quisultidine, ramixotidine, ranitidine, ranitidine bismuth citrate, ranitidine hydrochloride, roxatidine, sufotidine, tiotidine, tuvatidine, venritidine, xaltidine or a pharmaceutically acceptable salt, solvate, hydrate, enantiomer, pro-drug or polymorph thereof.

3. The pharmaceutical composition according to claim 2, the H2-receptor antagonists preferably famotidine or nizatidine or ranitidine or a pharmaceutically acceptable salt, solvate, hydrate, enantiomer, pro-drug or polymorph thereof.

4. The pharmaceutical composition according to claim 1 or 3, comprising dabigatran or a pharmaceutically acceptable salt, solvate, hydrate, enantiomer, pro-drug or polymorph thereof and famotidine or a pharmaceutically acceptable salt, solvate, hydrate, enantiomer, pro-drug or polymorph thereof.

5. The pharmaceutical composition according to claim 1 or 3, comprising dabigatran or a pharmaceutically acceptable salt, solvate, hydrate, enantiomer, pro-drug or polymorph thereof and nizatidine or a pharmaceutically acceptable salt, solvate, hydrate, enantiomer, pro-drug or polymorph thereof.

6. The pharmaceutical composition according to claim 1 or 3, comprising dabigatran or a pharmaceutically acceptable salt, solvate, hydrate, enantiomer, pro-drug or polymorph thereof and ranitidine or a pharmaceutically acceptable salt, solvate, hydrate, enantiomer, pro-drug or polymorph thereof.

7. The pharmaceutical composition according to any preceding claims, wherein the pharmaceutically acceptable salt, solvate, hydrate, enantiomer, pro-drug or polymorph of dabigatran is the etexilate or etexilate mesylate pro-drugs of dabigatran.

8. The pharmaceutical composition according to claim 4 or 7, wherein dabigatran etexilate or etexilate mesylate present in an amount of between 50 mg and 250 mg and famotidine or a pharmaceutically acceptable salt, solvate, hydrate, enantiomer, pro-drug or polymorph thereof is present in an amount of between 5 mg and 150 mg.

9. The pharmaceutical composition according to claim 5 or 7, wherein dabigatran etexilate or etexilate mesylate present in an amount of between 50 mg and 250 mg and nizatidine or a pharmaceutically acceptable salt, solvate, hydrate, enantiomer, pro-drug or polymorph thereof is present in an amount of between 10 mg and 500 mg.

10. The pharmaceutical composition according to claim 6 or 7, wherein dabigatran etexilate or etexilate mesylate present in an amount of between 50 mg and 250 mg and ranitidine or a pharmaceutically acceptable salt, solvate, hydrate, enantiomer, pro-drug or polymorph thereof is present in an amount of between 5 mg and 500 mg.

11. The pharmaceutical composition according to any preceding claims, wherein said pharmaceutical composition is formulated as tablets comprising compressed tablets, coated or uncoated tablets, multilayer tablets, buccal tablets, sublingual tablets, effervescent compositions, effervescent tablets, immediate release tablets, modified release tablets, film-coated tablets, orally disintegrating tablets, gastric disintegrating tablets, pills, capsules, hard or soft gelatin capsules, powders, mini tablets, pellets, coated bead systems, granules, microspheres, ion exchange resin systems, sterile solutions or suspensions, steril ocular solutions, aerosols, sprays, drops, ampoules, suppositories, ocular systems, parenteral systems, creams, gels, ointments, dragees, sachets; films, orally administrable films, solutions, solids; elixirs, tinctures, suspensions, syrups, colloidal dispersions, dispersions, emulsions and thereof.

12. The pharmaceutical composition according to any preceding claims for use in antithrombotic treatment and reducing the risk of stroke and systemic embolism in patients with non-vascular atrial fibrilation and protecting the gastrointestinal tract from side effects associated with antithrombotic therapy.

13. The pharmaceutical composition according to claim 11, wherein said pharmaceutical composition is formulated preferably in the form of tablet or capsule or multilayer tablet comprising etexilate or etexilate mesylate pellets of dabigatran and H2-receptor antagonist pellets.

14. The pharmaceutical composition according to claim 13, further comprising an inert barrier layer.

15. The pharmaceutical composition according to claim 14, wherein said inert barrier layer is selected from the group comprising starch, lactose, sugar alcohol, lowsubstituted hydroxypropyl cellulose, hydroxypropyl cellulose, hydroxypropyl methylcellulose, polyvinylpyrrolidone, polyvinyl alcohol, methylcellulose, hydroxyethyl methylcellulose or mixtures thereof.

16. The pharmaceutical composition according to any preceding claims, wherein the one or more pharmaceutically acceptable excipients are selected from the group comprising buffering agents, stabilizers, binders, diluents, dispersing agents, lubricants, glidants, disintegrants, plasticizers, preservatives, sweeteners, flavoring agents, coloring agents, acids or mixtures thereof.

17. The pharmaceutical composition according to claim 16, wherein said acid is selected from a group comprising organic acids or inorganic acids or mixtures thereof.

18. The pharmaceutical composition according to claim 17, wherein the organic acid is selected from a group comprising acetic acid, acrylic acid, adipic acid, aldaric acid, ascorbic acid, azelaic acid, benzoic acid, butyric acid, chloroacetic acid, citric acid, crotonic acid, dimercaptosuccinic acid, formic acid, fumaric acid, gallic acid, glutaric acid, itaconic acid, lactic acid, malic acid, maleic acid, malonic acid, monanoic acid, methacrylic acid, naphtenic acid, oleic acid, oxalic acid, palmitic acid, paracetic acid, propionic acid, pimelic acid, salicylic acid, sebacic acid, succinic acid, stearic acid, sorbic acid, tartaric acid, thiomalic acid, uric acid, glyceric acid, xylonic acid, gluconic acid, neuraminic acid, ulosonic acid, ketodeoxyoctulosonic acid, glucuronic acid, galacturonic acid, iduronic acid, meso-galactaric acid, glycolic acid, mandelic acid, aspartic acid, glutamic acid or mixtures thereof.

19. The pharmaceutical composition according to claim 17, wherein the inorganic acid is selected from a group comprising hydrogen halides and their solutions: hydrofluoric acid (HF), hydrochloric acid (HCl), hydrobromic acid (HBr), hydroiodic acid (HI); halogen oxoacids: hypochlorous acid (HClO), chlorous acid (HClO2), chloric acid (HClO3), perchloric acid (HClO4), and corresponding compounds for bromine and iodine; sulfuric acid (H2SO4), fluorosulfuric acid (HSO3F), nitric acid (HNO3), phosphoric acid (H3PO4), fluoroantimonic acid (HSbF6), fluoroboric acid (HBF4), hexafluorophosphoric acid (HPF6), chromic acid (H2CrO4), boric acid (H3BO3) or mixtures thereof.

20. The pharmaceutical composition according to claim 17, wherein said pharmaceutical composition is in the form of tablet or capsule or multilayer tablet comprising etexilate or etexilate mesylate of dabigatran pellets and H2-receptor antagonist pellets and acid pellets which are separate pellets or bilayer or multilayer pellets.

21. The pharmaceutical composition according to claim 20, wherein said dabigatran pellets comprising preferably methacrylic acid-ethyl acrylate copolymer or polyvinyl alcohol (PVA) or lactose monohydrate or sugar pellet or polyvinylpyrrolidone (PVP) or dibasic calcium phosphate (DCP) or mixtures thereof.

22. The pharmaceutical composition according to claim 20, wherein said acid pellets comprising preferably adipic acid or sorbic acid or succinic acid or glutaric acid or nitric acid and as an excipient xanthan gum or isopropyl alcohol (IPA) or sodium alginate or microcrystalline cellulose or mannitol or lactose or silicon dioxide or methacrylic acid-ethyl acrylate copolymer or polyvinyl alcohol (PVA) or mixtures thereof.

23. The pharmaceutical composition according to claim 20, wherein said H2-receptor antagonist pellets comprising preferably microcrystalline cellulose (MCC) or pregelatinized starch or hydroxypropyl cellulose or yellow iron oxide or croscarmellose sodium or sugar pellets or polyvinylpyrrolidone or mixtures thereof.
